# EUROPEAN PATENT SPECIFICATION

(11) **EP 1 701 933 B1**
(45) Date of publication and mention of the grant of the patent: **03.09.2008**
(21) Application number: 04807810.9
(22) Date of filing: 17.12.2004
(51) Int. Cl.: C07C 233/87, A61K 31/166, A61P 7/00, A61P 7/02, A61P 9/00, A61P 9/10, A61P 11/00, A61P 17/00, A61P 19/00, A61P 25/00, A61P 25/28, A61P 31/00, A61P 31/22, A61P 37/00, A61P 37/08

(54) **NOVEL COMPOUNDS**
NEUE VERBINDUNGEN
NOUVEAUX COMPOSES

(30) Priority: 20.12.2003 GB 0329584
(43) Date of publication of application: 20.09.2006
(73) Proprietor: Mitsubishi Tanabe Pharma Corporation, Chuo-ku Osaka-shi Osaka 541-8505 (JP)
(72) Inventor: WARD, Robert William, c/o GlaxoSmithKline p.l.c., Third Avenue, Harlow, Essex CM195AW (GB); PRADET, Charlotte, c/o GlaxoSmithKline p.l.c., Stevenage, Hertfordshire SG12NY (GB); ANDREWS, Ian Philip, c/o GlaxoSmithKline p.l.c., Stevenage, Hertfordshire SG12NY (GB)
(74) Representative: Vossius & Partner
(86) International application number: PCT/JP2004/019455
(87) International publication number: WO 2005/061440

(56) References cited:
- WO-A-99/36393
- US-A1- 2003 027 814

## Description

The present invention relates to novel compounds, processes for their preparation compositions comprising them and their use in the manufacture of a medicament for the treatment or prevention of certain diseases capable of being modulated by the inhibition of cell adhesion. More particularly the present invention relates to novel phenylalanine derivatives that inhibit α₄ integrin mediated cell adhesion and which are believed to be useful for the treatment or prevention of inflammatory disease.

The multiple adhesive interactions between leukocytes and endothelial cells or extracellular matrix proteins are a key factor in the regulation of immunity and inflammation. The earliest events in the migration of leukocytes out of the vasculature at site of inflammation include leukocyte rolling followed by changes in integrin avidity, which lead to subsequent firm adhesion (for reviews see Butcher, Cell 67:1033-1036 (1991); Harian, Blood 3:513-525 (1985); Hemler, Annu. Rev. Immunol. 8:365-400 (1890); Osbom, Cell 62:3-6 (1990); Shimizu et al., Immunol. Rev. 114:109-143 (1990); Springer, Nature 346:425-434 (1990); and Springer, Cell 76:301-314 (1994)). In - response to chemotactic factors, the leukocytes migrate through two adjacent endothelial cells and into tissues that are composed, in part, of the extracellular matrix protein fibronectin (FN) (see Wayner et al., J. Cell Biol. 105:1873-1884 (1987)) and collagen (CN) (see Bomstein et al., , Ann. Rev. Biochem. 49:957-1003 (1980); and Miller, Chemistry of the collagens and their distribution, in "Extracellular Matrix Biochemistry", K.A. Piez and A.H. Reddi, editors, Elsevier, Amsterdam, 41-78 (1983)). Important recognition molecules that participate in these adhesive reactions belong to the integrin gene superfamily (for reviews see Hemler, Annu. Rev. Immunol. 8:365-400 (1990); Hynes, Cell 48:549-554 (1987); Shimizu et al., Immunol. Rev. 114:109-143 (1990); and Springer, Nature 346:425-434 (1990)).

Integrins are heterodimers composed of non-covalently associated subunits, referred to as the alpha (α) and beta (β) subunits. To date, 8 integrin p subunits have been identified which can associate with 16 distinct α subunits to form at least 23 distinct

The α₄β₁ integrin, also known as VLA-4 (Very Late Antigen-4), is constitutively expressed on the surface of leukocytes including lymphocytes, monocytes, eosinophils and basophils (see Hemler et al., J. Bio. Chem. 262:11478-11485 (1987); and Bochner et al., J. Exp. Med. 173:1553-1556 (1991)). VLA-4 is reported to be present on neutrophils from septic patients (see Ibbotson et al., Nature Med. 7:465-470 (2001)). VLA-4 binds to vascular cell adhesion molecule-1 (VCAM-1) on activated endothelial cells, resulting in extravasation of leukocytes (Elices et al., Cell 60:577-584 (1990)). Once the cells have reached the extravascular space, VLA-4 can bind to the connecting segment 1 (CS-1), an alternatively spliced region of the FN A chain (Wayner et al., J. Cell Biol. 109:1321-1330 (1989)). In addition, VLA-4 is known to bind to osteopontin, a protein upregulated in arteriosclerotic plaques (see Bayless et al., J. Cell Science 111:1165-1174 (1998)).

The α₄β₇ integrin, also known as LPAM-1 (Lymphocyte-Peyer's patch Adhesion Molecule-1), interacts with three known ligands (VCAM-1, CS-1, MAdCAM-1). One ligand which shows unique specificity for α₄β₇ is Mucosal Addressin Cell Adhesion Molecule-1 (MAdCAM-1) (see Andrew et al., J. Immunol. 153:3847-3861 (1994); Briskin et al., Nature 363:461-464 (1993); and Shyjan et al., J. Immunol. 156:2851-2857 (1996)). MAdCAM-1 is highly expressed on Peyer's patch high endothelial venules, in mesenteric lymph nodes, and on gut lamina propria and mammary gland venules (Berg et al., Immunol. Rev. 108:5-18 (1989)). Integtin α₄β₇ and MAdCAM-1 have been shown to be important in regulating lymphocyte trafficking to normals intestine (Holzmann et al., Cell 56:37-46 (1989)).

Also, it has been described that an orally bioavailable, non-peptide small molecule antagonist of the α₄ integrins α₄β₁ and α₄β₇ could be useful in treating or preventing conditions such as asthma, inflammatory bowel disease, rheumatoid arthritis, multiple sclerosis and other diseases (see patent applications WO 99/36393 and WO 02/18320).

A novel class of compounds has now been discovered which fall within the generic scope of patent application WO 99/36393, but are not specifically disclosed therein.

The present invention therefore provides, in a first aspect, a compound of formula (I) or a pharmaceutically acceptable salt, ester or solvate thereof: 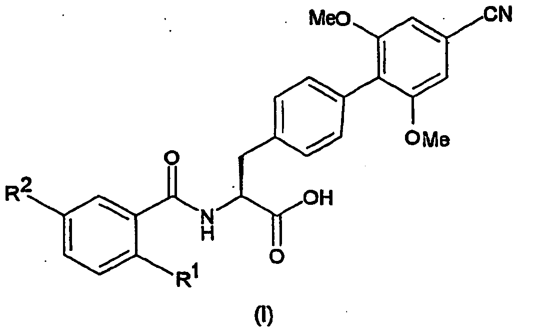 in which
R¹ is bromo; and
R² is halogen, C₁₋₆alkyl or C₁₋₆alkoxy.

Preferably R² is halogen or C₁₋₆alkoxy.

More preferably R² is fluoro, methoxy or ethoxy.

In a further aspect, the present invention provides E1-E7 (as described below) or a pharmaceutically acceptable derivative thereof, i.e.
(S)-2-{[1-(2-Bromo-5-ethoxyphenyl)methanoyl]amino}-3-(4'-cyano-2',6'-dimethoxybiphenyl-4-yl)propionic acid;
(S)-2-{[1-(2-Bromo-5-fluorophenyl)methanoyl]amino}-3-(4'-cyano-2',6'-dimethoxybiphenyl-4-yl)propionic acid;
(S)-2-{[1-(2-Bromo-5-methoxyphenyl)methanoyl]amino}-3-(4'-cyano-2',6'-dimethoxybiphenyl-4-yl)propionic acid;
(S)-2-{[1-(2-Bromo-5-methylphenyl)methanoyl]amino}-3-(4'-cyano-2',6'-dimethoxybiphenyl-4-yl)propionic acid);
(S)-2-{[(2-Bromo-5-chlorophenyl)methanoyl]amino}-3-[4'-cyano-2',6'-dimethoxy)biphenyl-4-yl]propionic acid;
(S)-2-{[(2,5-Dibromophenyl)methanoyl]amino}-3-[4'-cyano-2',6'-dimethoxy)biphenyl-4-yl]propionic acid;
(S)-2-{[(5-(*iso*-Propoxy)-2-bromophenyl)methanoyl]amino}-3-[4'-cyano-2',6'-dimethoxy)biphenyl-4-yl]propionic acid
or a pharmaceutically acceptable salt, ester or solvate thereof.

Throughout the present specification, unless otherwise stated:
the term "halogen" is used to describe a group selected from fluorine, chlorine, bromine or iodine;
the term "C₁₋₆alkyl" is used to describe a group or a part of the group comprising a linear or branched alkyl group containing from 1 to 6 carbon atoms; examples of such groups include methyl, ethyl, propyl, isopropyl, n-butyl, isobutyl, tert butyl, pentyl or hexyl;
the term "C₁₋₆alkoxy" is used to describe a group or a part of the group wherein an oxygen atom is bound to the above mentioned C₁₋₆alkyl group; examples of such groups include methoxy, ethoxy, propoxy, isopropoxy, n-butoxy, isobutoxy, tert butoxy, pentoxy or hexoxy.

The characteristics of the present compounds are the introduction of a cyano group at the 4'-position of the biphenyl nucleus in combination with the claimed 2,5-disubstituted benzoyl group.

The compounds of the formula (I) or a pharmaceutically acceptable salt, ester or solvate thereof have potent inhibitory activity against α₄ integrin mediated cell adhesion. Further, it has been found that certain examples show excellent bioavailability after oral administration and / or good systemic exposure.

E1, E2 and E3 (as described below) exhibit an advantageous combination of the above characteristics.

It will be appreciated that the compounds of formula (I) or a pharmaceutically acceptable salt, ester or solvate thereof may have more than one asymmetric carbon atoms and therefore may occur as diastereomers. All such isomeric forms are included within the present invention, including mixtures thereof.

Separation of diastereoisomers may be achieved by conventional techniques, e.g. by fractional crystallisation, chromatography or HPLC. A single stereoisomeric form of the compound may also be prepared from a corresponding optically pure intermediate or by resolution, such as HPLC of the corresponding racemate using a suitable chiral support or by fractional crystallisation of the diastereoisomeric salts formed by reaction of the corresponding racemate with a suitable optically active acid or base, as appropriate. Alternatively a mixture of enantiomers may be separated by chemical reaction with an appropriate chiral compound with the formation of a new covalently bonded species, for example the coupling of a racemic carboxylic acid with a chiral amine or alcohol to give a diastereomeric mixture (in the case of amides or esters respectively), which may be separated by conventional techniques such as column chromatography, HPLC or fractional crystallisation. The single diastereomers may then be converted to the single enantiomers of the desired compound by appropriate chemistry such as hydrolytic cleavage of the new covalent bond.

As used herein, the term "pharmaceutically-acceptable derivative", means any pharmaceutically acceptable salt, ester or solvate of a compound of the invention, which upon administration to the recipient is capable of providing (directly or indirectly) a compound of the invention, or an active metabolite or residue thereof. Such derivatives are recognisable to those skilled in the art, without undue experimentation. Nevertheless, reference is made to the teaching of Burger's Medicinal Chemistry and Drug Discovery, 5th Edition, Vol 1: Principles and Practice. Preferred pharmaceutically acceptable derivatives are salts and esters.

Those skilled in the art of organic chemistry will appreciate that many organic compounds can form complexes with solvents in which they are reacted or from which they are precipitated or crystallised. These complexes are known as "solvates". For example, a complex with water is known as a "hydrate". Solvates of the compound of the invention are within the scope of the invention.

As used herein, the term "prodrug" i.e. ester, means a compound which is converted within the body, e.g. by hydrolysis in the blood, into its active form that has medical effects. Pharmaceutically acceptable prodrugs are described in T. Higuchi and V. Stella, Prodrugs as Novel Delivery Systems, Vol. 14 of the A.C.S. Symposium Series, Edward B. Roche, ed., Bioreversible Carriers in Drug Design, American Pharmaceutical Association and Pergamon Press, 1987, and in D. Fleisher, S. Ramon and H. Barbra "Improved oral drug delivery: solubility limitations overcome by the use of prodrugs", Advanced Drug Delivery Reviews (1996) 19(2) 115-130.

Prodrugs are any covalently bonded carriers that release the compound of formula (I) or a pharmaceutically acceptable derivative thereof *in vivo* when such prodrug is administered to a patient Prodrugs are generally prepared by modifying functional groups in a way such that the modification is cleaved, either by routine manipulation or *in vivo,* yielding the parent compound. In the case of a carboxylic acid (-COOH), esters are employed, such as methyl esters, ethyl esters, double esters and the like. Esters may be active in their own right and /or be hydrolysable under *in vivo* conditions in the human body. Suitable pharmaceutically acceptable *in vivo* hydrolysable ester groups include those which break down readily in the human body to leave the parent add or its salt.

The compounds of the present invention may be in the form of and/or may be administered as a pharmaceutically acceptable salt For a review on suitable salts see Berge et al., J. Pharm. Sci., 1977, 66, 1-19.

Typically, a pharmaceutically acceptable salt may be readily prepared by using a desired acid or base as appropriate. The salt may precipitate from solution and be collected by filtration or may be recovered by evaporation of the solvent.

In the case of the compound of formula (I), suitable pharmaceutically acceptable salts are formed from pharmaceutically acceptable bases which include ammonium salts, alkali metal salts such as those of sodium and potassium, alkaline earth metal salts such as those of calcium and magnesium and salts with organic bases, including salts of primary, secondary and tertiary amines, such as isopropylamine, diethylamine, ethanolamine, trimethylamine, dicyclohexyl amine, N-methyl-D-glucamine and tris(hydroxymethyl)methylamine.

In a further aspect, the present invention also provides a process for the preparation of the compound of formula (I) which comprises hydrolyzing a carboxylic acid ester derivative of formula (II): 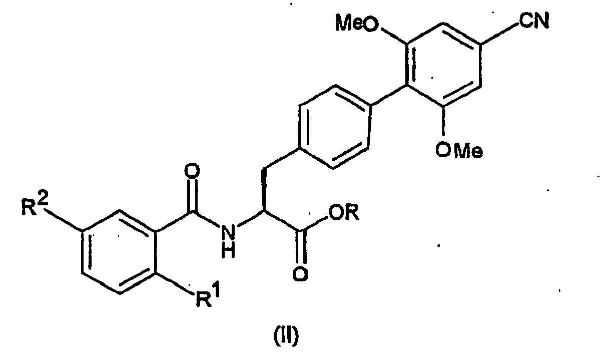 in which R¹ and R² are as defined in formula (I) and R is a C₁₋₆ alkyl group and optionally thereafter forming a pharmaceutically acceptable salt, ester or solvate thereof.

Examples of suitable R groups are methyl or t-butyl, preferably methyl. Hydrolysis may either occur via an acidic or an alkaline medium. An illustration of hydrolysis in an alkaline medium would be treating the compound of formula (II) with an alkali metal hydroxide in a suitable solvent, e.g., treatment with lithium hydroxide in aqueous tetrahydrofuran. An illustration of hydrolysis in an acidic medium would be treating the compound of formula (II) with a mineral acid in a suitable co-solvent at elevated temperature, e.g., treatment with 5N hydrochloric acid in dioxan at 60°C overnight. Such methods are familiar to those skilled in the art.

The compounds of formula (II) can be prepared by reacting a compound of formula (III) or an acid addition salt thereof: 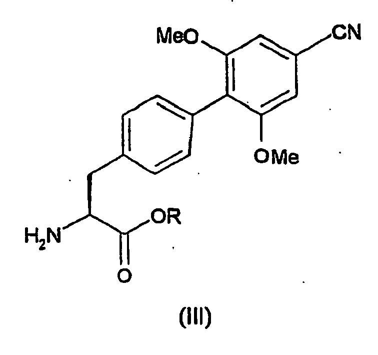 in which R is as defined in formula (II) with a compound of formula (IV): 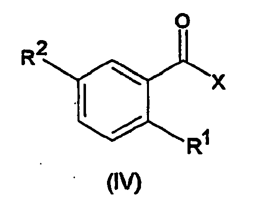 in which R¹ and R² are as defined in formula (I) and X is a hydroxy group or leaving group.

A suitable example of an acid addition salt of the compound of formula (III) is the hydrochloride. When X is a leaving group, a suitable example of a leaving group X is halogen, particularly chloro. Reactions between compounds of formula (III) and (IV) are typically carried out in an inert organic solvent such as tetrahydrofuran or dichloromethane or a mixed organic / aqueous system at ambient or elevated temperature in the presence of a suitable base, e.g., an organic base (such as triethylamine), an alkali metal carbonate (such as potassium carbonate) or a alkali metal hydrogen carbonate (such as sodium hydrogen carbonate). When X is a hydroxy group, reactions between compounds of formula (III) and (IV) are carried out using standard coupling methodology e.g. O-(7-azabenzatriazol-1-yl)-N,N,N',N'-tetramethyluronium hexafluorophosphate, triethylamine, in dry dimethylformamide stirred at room temperature overnight.

The compounds of formula (III) can be prepared by the coupling of a compound of formula (V): 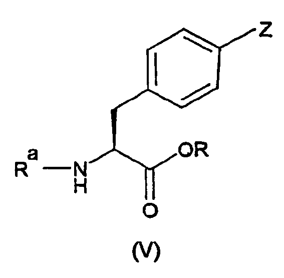 in which Z is a leaving group and R^{a} is a protecting group with a compound of formula (VI): 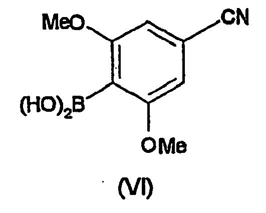 followed by removal of the protecting group R^{a}.

Suitable examples of leaving group Z are halogen, an alkanesulfonyloxy group (e.g., methanesulfonyl group), a haloalkanesulfonyloxy group (e.g., trifluoromethanesulfonyloxy group) or an arylsulfonyloxy group (e.g., p-toluenesulfonyloxy group). A suitable example of a protecting group R^{a} includes those listed below, particularly t-butyloxycarbonyl (Boc).

The compounds of formula (III) can also be prepared by the coupling of the compound of formula (V) with a compound of formula (VII): 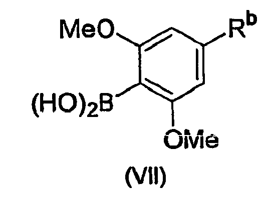 in which R^{b} is a hydroxymethyl group, followed by removal of the protecting group R^{a} and conversion of R^{b} to a cyano group. The reaction between the compounds of formula (V) and formula (VII) can be carried out by the similar manner to the reaction between the compounds of formula (V) and formula (VI). The protecting group of the resulting product can be removed by methods described above. The group R^{b} can be converted to a cyano group using procedures familiar to those skilled in the art and described herein.

The reaction between the compounds of formula (V) and formula (VI) or formula (VII) may, for example, be carried out under Suzuki coupling conditions which are familiar to those skilled in the art. By way of illustration, the reaction may be carried out using a palladium catalyst (e.g. tetrakis(triphenylphosphine)palladium(O)) in a suitable solvent (e.g., 1-methyl-2-pyrrolidone) at elevated temperature in the presence of a suitable base (e.g., triethylamine). The protecting group of the resulting product can be removed by methods familiar to those skilled in the art When the protecting group is t-butyloxycarbonyl the de-protection can be conducted by acid treatment (e.g., with hydrochloric acid) in an appropriate solvent (e.g., an alcohol such as ethanol or isopropanol).

The intermediate compound of formula (VI) can be prepared from commercially available 4-bromo-3,5-dimethoxybenzoic acid using procedures described herein. Intermediate compounds of formulae (II), (III) and (VI) are believed to be novel and form a yet further aspect of this invention.

The present invention provides the compound of formula (II). The present invention especially provides the compound of formula (II) in which R² is C₁₋₆ alkoxy or fluoro.

The present invention also provides the compound of formula (III) or an acid addition salt thereof.

The present invention further provides the compound of formula (VI).

Intermediate compounds (IV) and (V) are either commercially available or can be prepared using methods described herein, by methods known to those skilled in the art or by analogous methods thereto.

Those skilled in the art will appreciate that in the preparation of the compound of the formula (I) or a pharmaceutically acceptable salt, ester or solvate thereof, it may be necessary and/or desirable to protect one or more sensitive groups in the molecule to prevent undesirable side reactions. Suitable protecting groups for use according to the present invention are well known to those skilled in the art and may be used in a conventional manner. See, for example, "Protective groups in organic synthesis" by T.W. Greene and P.G.M. Wuts (John Wiley & Sons 1991) or "Protecting Groups" by P.J. Kocienski (Georg Thieme Verlag 1994). Examples of suitable amino protecting groups include acyl type protecting groups (e.g., formyl, trifluoroacetyl, acetyl), aromatic urethane type protecting groups (e.g., benzyloxycarbonyl (Cbz) and substituted Cbz), aliphatic urethane protecting groups (e.g., 9-fluorenylmethoxycarbonyl (Fmoc), t-butyloxycarbonyl (Boc), isopropyloxycarbonyl, cyclohexyloxycarbonyl) and alkyl type protecting groups (e.g., benzyl, trityl, chlorotrityl). Examples of suitable oxygen protecting groups may include for example alkyl silyl groups, such as trimethylsilyl or tert-butyldimethylsilyl; alkyl ethers such as tetrahydropyranyl or tert-butyl; or esters such as acetate.

Compounds of this invention may be tested for *in vitro* biological activity in accordance with the following assay.

### Jurkat J6 Scintillation Proximity Assay (SPA)

The Jurkat J6 Scintillation Proximity Assay was used to investigate the interaction of VLA-4 expressed on the Jurkat J6 cell membrane with test compounds. J6 cells (1 million cells/well) were allowed to coat wheat germ agglutinin coated SPA beads (Amersham, 1mg/well) in assay buffer containing 50mM HEPES, 100mM NaCl and 1mM MnCl₂ (pH adjusted to 7.5 with 4M NaOH). Tritiated ³H Standard Compound A (1-3 nM final assay concentration) and test compounds were dissolved in an appropriate solvent and diluted in assay buffer (the top assay concentration being 2.5µm; ten point dose response curve). Compounds were assayed in duplicate, a four parameter curve fit being applied. The equilibrium dissociation constant for each compound was calculated according to the method of Cheng & Prusoff (Biochem Pharmacol., 22(23) : 3099 - 3108 (1973)). Data were presented as the mean pKi.

Standard compound A is (2S)-3-[4-({[4-(aminocarbonyl)-1-piperidinyl]carbonyl}oxy)phenyl]-2-[((2S)-4-methyl-2-{[2-(2-methylphenoxy)acetyl]amino}pentanoyl)amino] propanoic acid potassium salt which is described in patent application WO 00/37444 (Glaxo Group Ltd. et al.). Tritiated ³H derivatives may be prepared employing conventional methods.

All examples prepared in accordance with this invention were tested in accordance with this procedure and were found to have a pKi ≥ 8.6.

Compounds of formula (I) or a pharmaceutically acceptable salt, ester or solvate thereof inhibit α₄ integrin mediated cell adhesion. It is believed that α₄ integrin mediated cell adhesion is implicated in a range of conditions such as rheumatoid arthritis (RA); asthma; allergic conditions such as rhinitis; adult respiratory distress syndrome; AIDS-dementia; Alzheimer's disease; cardiovascular diseases; thrombosis or harmful platelet aggregation; reocclusion following thrombolysis; reperfusion injury; skin inflammatory diseases such as psoriasis, eczema, contact dermatitis and atopic dermatitis; diabetes (e.g., insulin-dependent diabetes mellitus, autoimmune diabetes); multiple sclerosis; systemic lupus erythematosus (SLE); inflammatory bowel disease such as ulcerative colitis, Crohn's disease (regional enteritis) and pouchitis (for example, resulting after proctocolectomy and ileoanal anastomosis); diseases associated with leukocyte infiltration to the gastrointestinal tract such as Celiac disease, nontropical Sprue, enteropathy associated with seronegative arthropathies, lymphocytic or collagenous colitis, and eosinophilic gastroenteritis; diseases associated with leukocyte infiltration to other epithelial lined tissues, such as skin, urinary tract, respiratory airway, and joint synovium; pancreatitis; mastitis (mammary gland); hepatitis; cholecystitis; cholangitis or pericholangitis (bile duct and surrounding tissue of the liver); bronchitis; sinusitis; inflammatory diseases of the lung which result in interstitial fibrosis, such as hypersensitivity pneumonitis; collagen disease (in SLE and RA); sarcoidosis; osteoporosis; osteoarthritis; atherosclerosis; neoplastic diseases including metastasis of neoplastic or cancerous growth; wound (wound healing enhancement); certain eye diseases such as retinal detachment, allergic conjunctivitis and autoimmune uveitis; Sjogren's syndrome; rejection (chronic and acute) after organ transplantation; host vs. graft or graft vs. host diseases; intimal hyperplasia; arteriosclerosis (including graft arteriosclerosis after transplantation); reinfarction or restenosis after surgery such as percutaneous transluminal coronary angioplasty (PTCA) and percutaneous transluminal artery recanalization; nephritis; tumor angiogenesis; malignant tumor; multiple myeloma and myeloma-induced bone resorption; sepsis; and central nervous system injury such as stroke, traumatic brain injury and spinal cord injury and Meniere's disease.

The compounds of the present invention can be preferably used for the preparation of a medicament for the treatment or prevention of asthma, allergic conditions such as rhinitis, inflammatory bowel disease such as ulcerative colitis and Crohn's disease, rheumatoid arthritis, atopic dermatitis, multiple sclerosis and rejection after organ transplantation. In particular, the compounds of the present invention can be used to treat or prevent inflammatory bowel disease or multiple sclerosis.

The compounds of the present invention can further be used for the preparation of a medicament for the treatment or prevention of conditions in which an inhibitor of α₄ integrin mediated cell adhesion is beneficial i.e. the aforementioned conditions.

The present invention also provides the compound of formula (I) or a pharmaceutically acceptable salt, ester or solvate thereof for use in therapy, particularly the treatment or prevention of the aforementioned disorders.

While it is possible for the compounds of the present invention to be administered alone, it is preferable to formulate into a pharmaceutical composition in accordance with standard pharmaceutical practice. Thus the invention also provides a pharmaceutical composition which comprises a therapeutically effective amount of the compound of formula (I) or a pharmaceutically acceptable salt, ester or solvate thereof in admixture with a pharmaceutically acceptable carrier or diluent.

The invention further provides a pharmaceutical composition comprising the compound of formula (I) or a pharmaceutically acceptable salt, ester or solvate thereof together with another therapeutically active agent.

There is further provided by the present invention a process of preparing a pharmaceutical composition, which process comprises mixing at least one compound of the invention or a pharmaceutically acceptable salt, ester or solvate thereof, together with a pharmaceutically acceptable carrier or diluent

The pharmaceutical compositions may be for human or animal usage in human and veterinary medicine and will typically comprise any one or more of a pharmaceutically acceptable diluent, carrier or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The carrier or diluent must be acceptable in the sense of being not deleterious to the recipient thereof. The pharmaceutically acceptable carrier or diluent may be, for example, binders (e.g., syrup, gum arabic, gelatin, sorbitol, tragacanth, polyvinylpyrrolidone), excipients (e.g., lactose, sucrose, corn starch, potassium phosphate, sorbitol, glycine), lubricants (e.g., magnesium stearate, talc, polyethylene glycol, silica) disintegrators (e.g., potato starch), wetting agents (e.g., sodium laurylsulfate), and the like.

The routes for administration (delivery) of the composition of the invention include, but are not limited to, one or more of: oral (e.g., as a tablet, capsule, or as an ingestible solution), topical, mucosal (e. g., as a nasal spray or aerosol for inhalation), nasal, parenteral (e. g., by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, epidural, sublingual.

For example, the compound can be administered orally in the form of tablets, capsules, ovules, elixirs, solutions or suspensions, which may contain flavouring or colouring agents, for immediate-, delayed-, modified-, sustained-, pulsed- or controlled-release applications. The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

The compounds of the invention may be milled using known milling procedures such as wet milling to obtain a particle size appropriate for tablet formation and for other formulation types. Finely divided (nanoparticulate) preparations of the compounds of the invention may be prepared by processes known in the art, for example see International Patent Application No. WO 02/00196 (SmithKline Beecham).

If the compound of the present invention is administered parenterally, then examples of such administration include one or more of: intravenously, intraarterially, intraperitoneally, intrathecally, intraventricularly, intraurethrally, intrastemally, intracranially, intramuscularly or subcutaneously administering the agent; and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

As indicated, the compound of the present invention can be administered intranasally or by Inhalation and is conveniently delivered in the form of a dry powder inhaler or an aerosol spray presentation from a pressurised container, pump, spray or nebuliser with the use of a suitable propellant, e. g. dichlorodifluoromethane, trichlorofluoromethane, dichlorotetrafluoroethane, a hydrofluoroalkane such as 1,1,1,2-tetrafluoroethane (HFA 134AT"") or 1, 1, 1, 2, 3, 3,-heptafluoropropane (HFA 227EA) (for example from Ineos Fluor), carbon dioxide or other suitable gas. In the case of a pressurised aerosol, the dosage unit may be determined by providing a valve to deliver a metered amount The pressurised container, pump, spray or nebuliser may contain a solution or suspension of the active compound, e. g., using a mixture of ethanol and the propellant as the solvent, which may additionally contain a lubricant, e. g. sorbitan trioleate. Capsules and cartridges (made, for example, from gelatin) for use in an inhaler or insufflator may be formulated to contain a powder mix of the compound and a suitable powder base such as lactose or starch.

Alternatively, the compound of the present invention can be administered in the form of a suppository or pessary, or it may be applied topically in the form of a gel, hydrogel, lotion, solution, cream, ointment or dusting powder. The compound of the present invention may also be dermally or transdermally administered, for example, by the use of a skin patch. They may also be administered by the pulmonary or rectal routes. They may also be administered by the ocular route. For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the agent of the present invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, it can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compositions of the present invention may be administered by direct injection.

In a preferred embodiment, the agents of the present invention are delivered systemically (such as orally, buccally, sublingually), more preferably orally.

Hence, preferably the agent is in a form that is suitable for oral delivery.

Typically, a physician will determine the actual dosage which will be most suitable for an individual subject. The specific dose level and frequency of dosage for any particular individual may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

For oral and parenteral administration to humans, the daily dosage level of the agent may be in single or divided doses.

A proposed dose of the compounds according to the present invention for administration to a human (of approximately 70kg body weight) is 0.1 mg to 2g, more typically 0.1 mg to 1g, of the active ingredient per unit dose, expressed as the weight of free acid. The unit dose may be administered, for example, 1 to 4 times per day. The dose will depend on the route of administration. It will be appreciated that it may be necessary to make routine variations to the dosage depending on the age and weight of the patient as well as the severity of the condition. The dosage will also depend on the route of administration. The predse dose and route of administration will ultimately be at the discretion of the attendant physician or veterinarian.

The compounds of the invention may also be used in combination with other therapeutic agents. The invention thus provides, in a further aspect, a combination comprising a compound of the invention or a pharmaceutically acceptable salt, ester or solvate thereof together with a further therapeutic agent

When a compound of formula (I) or a pharmaceutically acceptable salt, ester or solvate thereof is used in combination with a second therapeutic agent active against the same disease state the dose of each compound may differ from that when the compound is used alone. Appropriate doses will be readily appreciated by those skilled in the art. It will be appreciated that the amount of a compound of the invention required for use in treatment or prevention will vary with the nature of the condition and the age and the condition of the patient and will be ultimately at the discretion of the attendant physician or veterinarian. Examples of other active agents that may be combined with the compounds of formula (I) or of pharmaceutically acceptable salt, ester or solvate thereof include, but not limited to: (a) other VLA-4 antagonists; (b) H1 histamine antagonists; (c) NSAID's; (d) anti-diabetic agent e.g. glitazones (e) anti-cholinergic agents (f) COX-2 inhibitors e.g. 2-(4-ethoxy-phenyl)-3-(4-methanesulfonyl-phenyl)-pyrazolo[1,5-b]pyridazine (as disclosed in patent application WO 99/12930); (g) PDE-IV inhibitors; (h) steroids e.g. corticosteroids; (i) beta agonists; (j) antgonists of the chemokine receptors e.g. CCR-2, CCR-3, CCR-5 and CCR-8; (k) suitable multiple sclerosis treatments or preventions such as interferon; (I) LFA-1 antagonists; (m) TNF inhibitors; (n) Sulphasalazine and 5-aminosalicylates and (o) Immunosuppressants.

The combinations referred to above may conveniently be presented for use in the form of a pharmaceutical formulation and thus pharmaceutical formulations comprising a combination as defined above together with a pharmaceutical acceptable carrier or excipient comprise a further aspect of the invention. The individual components of such combinations may be administered either sequentially or simultaneously in separate or combined pharmaceutical formulations by any convenient route. When administration is sequential, either the compound of the invention or the second therapeutic agent may be administered first When administration is simultaneous, the combination may be administered either in the same or different pharmaceutical composition.

When combined in the same formulation it will be appreciated that the two compounds must be stable and compatible with each other and the other components of the formulation. When formulated separately they may be provided in any convenient formulation, conveniently in such manner as are known for such compounds in the art.

The following Preparations and Examples illustrate the preparation of compounds of the invention.

### General Method

where so indicated in the experimental section the term MDAP represents Mass Directed Auto Preparation, an automated system for compound purification by preparative HPLC with detection and collection by desired mass through use of a mass spectrometer in combination with a preparative HPLC system. In the following, a Waters FractionLynx MDAP system was employed with an appropriate reverse phase column using a water / acetonitrile gradient, both solvents containing 0.1% formic acid.

### Preparation 1

### (S)-2-tert-Butoxycarbonylamino-3-(4-hydroxyphenyl)propionic acid methyl ester (P1)

Di-*tert*-butyl dicarbonate (200 g, 0.92 mol) was added portionwise to a mixture of L-tyrosine methyl ester hydrochloride (200 g, 0.86 mol, Aldrich) and sodium hydrogen carbonate (100 g, 1.19 mol) in dichloromethane (1 L) and water (1 L). The mixture was stirred for 2 hours at room temperature. The organic layer was separated and the aqueous phase re-extracted with dichloromethane (500 mL). The combined organic layers were dried over magnesium sulfate, filtered and concentrated under reduced pressure to yield the crude title compound as a colourless glass, which was used in the following step without purification; LC/MS (ES-ve): [M-H]- at m/z 294 (C₁₅H₂₁NO₅ requires[M-H]⁻ at m/z 294).

### Preparation 2

### (S)-2-tert-Butoxycarbonylamino-3-(4-trifluoromethanesulfonyloxyphenyl)propionic acid methyl ester (P2)

Pyridine (58 mL, 0.72 mol) was added to a solution of crude (S)-2-*tert-*butoxycarbonylamino-3-(4-hydroxyphenyl)propionic acid methyl ester (P1, 70.5 g, 0.24 mol) in dichloromethane (1 L) under argon. The solution was cooled in ice, and then trifluoromethanesulfonic anhydride (52 mL, 0.31 mol) was added dropwise with stirring. After the addition was completed, the mixture was stirred in ice for 2 hours, washed with 2M hydrochloric acid (500 mL), dried over magnesium sulfate, filtered and concentrated under reduced pressure. The crude product was purified by silica gel chromatography (Biotage 75L, 800 g silica) eluting with ethyl acetate:hexane (15:85) to yield the title compound as a colourless oil which slowly solidified to give a white solid; LC/MS (ES+ve): [M-BOC+H]⁺ at m/z 328 (C₁₆H₂₀NO₇S requires [M+H]⁺ at m/z 428).

### Preparation 3

### 4-Hydroxymethyl-2,6-dimethoxyphenylboronic acid (P3)

A solution of (3,5-dimethoxyphenyl)methanol (53 g, 0.31 mol, Aldrich) in dry tetrahydrofuran (1 L) was cooled to -50°C to -70°C under argon and treated with n-butyllithium (450 mL, 1.6M in hexane, 0.72 mol) over 30 minutes. After the addition, the reaction mixture was allowed to warm to 0°C over 45 minutes and then left to stand at room temperature for 2 hours. The reaction was subsequently re-cooled to -60°C and treated with trimethylborate (135 mL, 1.15 mol) portionwise. Following the addition, the mixture was allowed to warm to room temperature and stirred for a further 18 hours. The reaction was quenched at 0°C by the portionwise addition of an aqueous solution of citric acid (75 g of citric acid in 300 mL of water). The aqueous layer was saturated by the addition of sodium chloride and the product extracted with ethyl acetate (2 x 1L). The combined organic layers were dried over magnesium sulfate, filtered and concentrated under reduced pressure. Ethyl acetate (100 mL) was added to the residue and the resulting colourless precipitate collected by filtration and dried at 40°C under vacuum to yield the title compound as a colourless solid which was used in the following reaction without purification.

### Preparation 4

### (S)-2-tert-Butoxycarbonylamino-3-(4'-hydroxymethyl-2',6'-dimethoxybiphenyl-4-yl)propionic acid methyl ester (P4)

Triethylamine (28 mL, 0.20 mol) was added to a solution of (S)-2*-tert* butoxycarbonylamino-3-(4-trifluoromethanesulfonyloxyphenyl)propionic acid methyl ester (P2, 42.73 g, 0.10 mol) and 4-hydroxymethyl-2,6-dimethoxyphenyl boronic acid (P3, 29.7 g, 0.14 mol) in dry dimethylformamide (250 mL). After degassing with argon, tetrakis(triphenylphosphine)palladium(0) (5.8 g, 5 mmol) was added and the mixture was heated at 90°C for 1 hour under argon. [NB A slight exotherm was observed]. After allowing to cool to room temperature, the mixture was diluted with ethyl acetate (1 L) and water (700 mL), and separated. The organic layer was washed with water (2 x 300 mL), dried over magnesium sulfate, filtered and concentrated under reduced pressure. The crude product was purified by silica gel chromatography (Biotage 75L, 800 g silica) eluting with ethyl acetate:hexane (50:50) to yield the title compound as a colourless solid; LC/MS (ES+ve): [M-BOC+H]⁺ at m/z 346 (C₂₄H₃₁NO₇ requires [M+H]⁺ at m/z 446).

### Preparation 5

### (S)-2-tert-Butoxycarbonylamino-3-(4'-formyl-2',6'-dimethoxybiphenyl-4-yl)propionic acid methyl ester (P5)

Manganese dioxide (230 g, 2.64 mol) was added portionwise to a solution of (S)-2*-tert-*butoxycarbonylamino-3-(4'-hydroxymethyl-2',6'-dimethoxybiphenyl-4-yl)propionic acid methyl ester (P4, 28.98 g, 65.1 mmol) in dichloromethane (1L). The resulting suspension was stirred at room temperature for 1 hour and then filtered through celite, washing the celite pad with further dichloromethane (1 L). The filtrate was concentrated at reduced pressure to yield the product as a colourless foam which was used without further purification; LC/MS (ES+ve): [M-BOC+H]⁺ at m/z 344 (C₂₄H₂₉NO₇ requires [M+H]⁺ at m/z 444).

### Preparation 6

### (S)-2-tert-Butoxycarbonylamino-3-[4'-(hydroxyiminomethyl)-2',6'-dimethoxybiphenyl-4-yl]propionic acid methyl ester (P6)

Hydroxylamine hydrochloride (7.4 g, 106 mmol) and diisopropylethylamine (18 mL, 106 mmol) were added to a solution of (S)-2-tert-butoxycarbonylamino-3-(4'-formyl-2',6'-dimethoxybiphenyl-4-yl)propionic acid methyl ester (P5, 23.6 g, 53 mmol) in tetrahydrofuran (300 mL). The reaction mixture was heated at reflux for 2 hours and then allowed to cool to room temperature. The solution was concentrated at reduced pressure and then redissolved in ethyl acetate (500 mL), washed with 10% aqueous citric acid, water and brine (500 mL each), dried over magnesium sulfate, filtered and concentrated under reduced pressure to yield a colourless foam, which was used in the next step without further purification; LC/MS (ES+ve): [M-BOC+H]⁺ at m/z 359 (C₂₄H₃₀N₂O₇ requires [M+H]⁺ at m/z 459).

### Preparation 7

### (S)-2-Amino-3-(4'-cyano-2',6'-dimethoxybiphenyl-4-yl)propionic acid methyl ester hydrochloride (P7)

Thionyl chloride (30 mL, 411 mmol) was added dropwise to a solution of (S)-2*-tert-*butoxycarbonylamino-3-[4'-(hydroxyiminomethyl)-2',6'-dimethoxybiphenyl-4-yl]propionic acid methyl ester (P6, 46.5 g, 101 mmol) in dichloromethane (500 mL) at 0°C under argon. The reaction was allowed to warm to room temperature and stirred for 3 days. The precipitated solid was collected by filtration, washed with dichloromethane (200 mL) and dried at 45°C under reduced pressure. The title compound was isolated as a white solid; LC/MS (ES+ve): [M+H]⁺ at m/z 341 (C₁₉H₂₀N₂O₄ requires [M+H]⁺ at m/z 341).

### Preparation 8

### 2-Bromo-5-hydroxybenzoic acid methyl ester (P8)

2-Bromo-5-methoxybenzoic acid (30.0 g, 130 mmol, Aldrich) was stirred under argon in dry dichloromethane (600 mL), cooled in Dry Ice / acetone, as boron tribromide (1M in dichloromethane, 280 mL, 280 mmol) was added over 15 minutes. The mixture was then stirred at ambient temperature for 2.5 hours, yielding a precipitate. Methanol (300 mL) was then cautiously added dropwise (CARE: initially strongly exothermic, and with effervescence) over 30 minutes, finally giving a dark homogeneous solution, to which was added concentrated sulphuric acid (15 mL). The solution was stirred at reflux for 1 hour, cooled, and concentrated under reduced pressure. The residue was dissolved in dichloromethane (500 mL), washed with brine (500 mL), dried over magnesium sulfate, filtered and concentrated at reduced pressure to yield the title compound as a solid; LC/MS (ES+ve): [M+H]⁺ at m/z 231, 233 (C₈H₇BrO₃ requires [M+H]⁺ at m/z 231, 233).

### Preparation 9

### 2-Bromo-5-ethoxybenzoic acid methyl ester (P9)

Sodium hydride (60% in mineral oil, 4.24 g, 106 mmol) was stirred under argon in dry dimethylformamide (150 mL), cooling in ice, as 2-bromo-5-hydroxybenzoic acid methyl ester (P8, 20.41 g, 88 mmol) was added in dry dimethylformamide (150 mL) over 15 minutes. The mixture was stirred at ambient temperature for 45 minutes, re-cooled in ice, and treated with iodoethane (8.5 mL, 106 mmol). This mixture was stirred at ambient temperature for 3 hours, concentrated at reduced pressure, diluted with ethyl acetate (400 mL), washed with water (3 x 400 mL) and brine (400 mL), dried over magnesium sulfate, filtered and concentrated at reduced pressure to yield the title compound, contaminated with a trace of the corresponding ethyl ester, and residual mineral oil. This material was used in the next step without further purification; LC/MS (ES+ve): [M+H]⁺ at m/z 259, 261 (C₁₀H₁₁BrO₃ requires [M+H]⁺ at m/z 259, 261).

### Preparation 10

### 2-Bromo-5-ethoxybenzoic acid (P10)

2-Bromo-5-ethoxybenzoic acid methyl ester (P9, 20.53 g, 79 mmol) was stirred in tetrahydrofuran (600 mL), and treated with lithium hydroxide (18.2 g, 791 mmol) in water (200 mL). The mixture was stirred for 4 days, concentrated at reduced pressure, diluted with water (500 mL), acidified with 5N hydrochloric acid, and extracted into ethyl acetate (2 x 300 mL). The combined extracts were washed with brine (250 mL), dried over magnesium sulfate, filtered and concentrated at reduced pressure to give an off-white solid. Trituration with hexane, filtration and drying gave the title compound as a white powder; LC/MS (ES+ve): [M+H]⁺ at m/z 245, 247 (C₉H₉BrO₃ requires [M+H]⁺ at m/z 245, 247).

### Preparation 11

### (S)-2-{[1-(2-Bromo-5-ethoxyphenyl)methanoyl]amino}-3-(4'-cyano-2',6'-dimethoxybiphenyl-4-yl)propionic acid methyl ester (P11)

(S)-2-Amino-3-(4'-cyano-2',6'-dimethoxybiphenyl-4-yl)propionic acid methyl ester hydrochloride (P7, 37.1 g, 98.5 mmol) was dissolved in dichloromethane (500 mL) and water (400 mL) and cooled to 0°C under argon. Sodium hydrogen carbonate (20.1 g, 239.3 mmol) was added to the reaction mixture followed by the dropwise addition of 2-bromo-5-ethoxybenzoyl chloride (27.2 g, 103.7 mmol) {prepared from 2-bromo-5-ethoxybenzoic acid (P10) by standard procedures using oxalyl chloride (4 equiv.) in dihloromethane and a drop of dimethylformamide}. The reaction was stirred at 0°C for 1 hour and was then diluted with a saturated aqueous solution of sodium hydrogen carbonate (200 mL). After separation of the organic layer, the aqueous layer was re-extracted with dichloromethane (2 x 400 mL). The combined organic layers were dried over sodium sulfate, filtered and concentrated at reduced pressure. The product was purified by silica gel chromatography (Biotage 75L, 800 g silica) eluting with ethyl acetate: dichloromethane (3:97) to yield the title compound as a colourless solid; MS (ES+ve): [M+H]⁺ at m/z 567, 569 (C₂₈H₂₇BrN₂O₆ requires [M+H]⁺ at m/z 567, 569).

### Preparation 12

### (S)-2-{[1-(2-Bromo-5-methoxyphenyl)methanoyl]amino}-3-(4'-cyano-2',6'-dimethoxybiphenyl-4-yl)propionic acid ethyl ester (P12)

2-Bromo-5-methoxybenzoic acid (0.355 g, 1.54 mmol, Aldrich), O-(7-azabenzotriazol-1-yl)-N,N,N',N'-tetramethyluronium hexafluorophosphate (1.168 g, 2 equiv.), and triethylamine (1.069 mL, 5 equiv.) were added to dimethylformamide (25 mL) under argon with stirring at room temperature. After 0.5 hour the ethyl ester corresponding to P7 (P13), (S)-2-amino-3-(4'-cyano-2',6'-dimethoxybiphenyl-4-yl)propionic acid ethyl ester hydrochloride, (0.6 g, 1 equiv.) was added and stirring continued overnight. The solvent was then evaporated under reduced pressure and the residue partitioned between ethyl acetate and water. The organic layer was washed with water (x 2) and saturated aqueous sodium bicarbonate before evaporation to dryness. The crude product was purified by column chromatography on silica with a gradient of 0-10% methanol in dichloromethane and subsequently by preparative HPLC to give the title compound.
MS (AP+ve): [M+H]⁺ at m/z 567, 569 (C₂₈H₂₇BrN₂O₆ requires [M+H]⁺ at m/z 567, 569).

### Preparation 13

### (S)-2-Amino-3-(4'-cyano-2',6'-dimethoxybiphenyl-4-yl)propionic acid ethyl ester hydrochloride (P7 - corresponding ethyl ester) (P13)

Thionyl chloride (7.71 mL, 105.7 mmol) was added dropwise over 15 minutes to a solution of (S)-2-tert-butoxycarbonylamino-3-[4'-(hydroxyiminomethyl)-2',6'-dimethoxybiphenyl-4-yl]propionic acid ethyl ester (12.5 g, 26.5 mmol) [prepared analogously to the sequence P1-P6 except commencing in Preparation 1 with L-tyrosine ethyl ester hydrochloride (from Bachem) instead of L-tyrosine methyl ester hydrochloride] in dichloromethane (250 mL) at 0°C under argon. The reaction stirred for a further 15 minutes at batch temperature and then allowed to warm to room temperature and stirring continued overnight. The precipitated title compound was collected by filtration, washed with dichloromethane (2x15 mL) and dried under reduced pressure. LC/MS (ES+ve): [M+H]⁺ at m/z 355 (C₂₀H₂₂N₂O₄ requires [M+H]⁺ at m/z 355).

### Example 1

### (S)-2-{[1-(2-Bromo-5-ethoxyphenyl)methanoyl]amino}-3-(4'-cyano-2',6'-dimethoxybiphenyl-4-yl)propionic acid (E1)

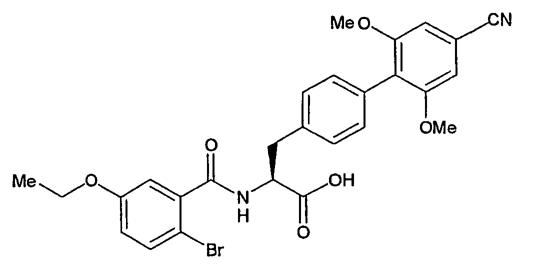

A solution of (S)-2-{[1-(2-bromo-5-ethoxyphenyl)methanoyl]amino}-3-(4'-cyano-2',6'-dimethoxybiphenyl-4-yl)propionic acid methyl ester (P11, 51.5 g, 90.8 mmol) in tetrahydrofuran (950 mL) was cooled to 0°C and treated with 0.5M aqueous lithium hydroxide (700 mL). The reaction mixture was stirred at 0°C for 1 hour and then acidified with 5M hydrochloric acid. The tetrahydrofuran was evaporated *in vacuo* and the residue was diluted with ethyl acetate. After separation of the organic layer, the aqueous phase was re-extracted with ethyl acetate. The combined organic layers were washed with water (x 2) and then dried over magnesium sulfate and the solvent was evaporated *in vacuo* to yield the title compound as a colourless solid;
¹H NMR δ (DMSO-d6): 1.29 (3H, t, *J* 7.0Hz), 2.96 (1H, dd, *J* 13.9, 10.9Hz), 3.22 (1H, dd, *J* 14.1, 4.2Hz), 3.71 (6H, s), 3.99 (2H, q, *J* 7.0Hz), 4.65 (1 H, ddd, *J* 10.9, 8.4, 4.3Hz), 6.69 (1 H, d, *J* 3.0Hz), 6.91 (1 H, dd, *J* 8.8, 3.1 Hz), 7.14 (2H, d, *J* 8.1 Hz), 7.24 (2H, s), 7.32 (2H, d, *J* 8.1Hz,), 7.47 (1 H, d, *J* 8.8Hz), 8.77 (1H, br. d, *J* 8.4Hz), 12.83 (1 H, br. s); MS (ES+ve) [M+H]⁺ at m/z 553, 555 (C₂₇H₂₅BrN₂O₆ requires [M+H]⁺ at m/z 553,555).

### Example 1 - alternative synthetic procedure

The title compound was also prepared using the following procedure.

### 4-Bromo-3,5-dimethoxybenzamide

Thionyl chloride (60 mL, 0.82 mol) was added to a stirred suspension of 4-bromo-3,5-dimethoxybenzoic acid (from Jintan Baocheng, 100 g, 0.38 mol) in toluene (700 mL) and dimethylformamide (1 mL). The reaction was stirred at reflux under nitrogen. HPLC after 2 hours showed complete consumption of acid to give methyl ester (from methanol quench of acid chloride). The volatiles were removed by distillation and the resulting solid added to 0.88 ammonia (350 mL). The mixture was stirred at ambient temperature for 45 minutes. Product was filtered, washed with water (2 x 200 mL) and dried under vacuum overnight to yield the title compound as an off-white solid.
¹H NMR δ (DMSO-d6): 3.89 (6H, s), 7.23 (2H, s), 7.52 (1 H, s), 8.2 (1 H, s)
MS (ES+ve) [M+H]⁺ at m/z 260, 262 (C₉H₁₀BrNO₃ requires [M+H]⁺ at m/z 260, 262)

### 4-Bromo-3,5-dimethoxybenzonitrile

Trifluoroacetic anhydride (129 mL, 0.91 mol) was added to a suspension of 4-bromo-3,5-dimethoxybenzamide (104 g, 0.38 mol) in pyridine (127 mL, 1.57 mol) and tetrahydrofuran (950 mL). The temperature was maintained at 20-25°C with an icebath. The orange/brown solution was stirred at 20-25°C under nitrogen for 1 hour. HPLC showed complete consumption of 4-bromo-3,5-dimethoxybenzamide to give product. The reaction mixture was concentrated to approximately 50% of the initial volume. Water (900 mL) was added and product filtered off, washed with water (2 x 250 mL) and dried at 40°C under vacuum to yield the title compound as a fluffy off-white solid.
¹H NMR δ (CDCl₃): 3.96 (6H, s), 6.8 (2H, s)

### 4-Cyano-2,6-dimethoxyphenylboronic acid

Isopropyl magnesium chloride (2M solution in tetrahydrofuran, 206 mL, 0.412 mol) was added to a slurry of 4-bromo-3,5-dimethoxybenzonitrile (50 g, 0.207 mol) in tetrahydrofuran (400 mL) at 5-10°C under nitrogen. The resulting solution was allowed to warm to ambient. HPLC analysis indicated complete consumption of starting material. The reaction mixture was cooled to 5-10°C and added to a solution of trimethylborate (103 mL, 0.92 mol) in tetrahydrofuran (100 mL), maintaining the temperature below 10°C during the addition. The reaction was allowed to warm to ambient temperature. HPLC showed complete reaction to product. The reaction mixture was cooled to 5-10°C and 1 M hydrochloric acid (500 mL) added, maintaining the temperature below 10°C during the addition. The mixture was stirred at ambient temperature for 30 minutes and then volatiles were removed by distillation. Product was filtered, washed with water and dried at 40°C under vacuum to yield the title compound as an off-white solid.
¹H NMR δ (DMSO-d₆): 3.78 (6H, s), 7.0 (2H, s), 9.25 (2H, s)

### (S)-2-Amino-3-(4'-cyano-2',6'-dimethoxybiphenyl-4-yl)propionic acid methyl ester hydrochloride

A solution of di-*tert*-butyldicarbonate (17.9 g, 81.8 mmol) in toluene (60 mL) was added dropwise to a solution of (S)-methyl tyrosine (from Flamma, 15.2 g, 77.9 mmol) in toluene (120 mL) at 90°C. The reaction mixture was stirred at 90°C for at least 30 minutes. Once complete by HPLC, the reaction mixture was cooled to 0°C and pyridine (19 mL, 0.23 mol) added keeping the temperature at 0°C. Trifluoromethanesulfonic anhydride (16.7 mL) was added dropwise keeping the temperature at 0°C. The orange slurry was stirred at 0°C for at least 2 hours. Once complete by HPLC 2M hydrochloric acid (133 mL) was added keeping the temperature at 0°C. The layers were separated and the upper organic layer washed with 10%w/w aqueous sodium carbonate solution (138 mL) and 36%w/w brine (138 mL). The organic layer was concentrated to approximately 60 mL and solid sodium chloride (30 g) added, followed by n-heptane (300 mL). The mixture was filtered and the filtrate extracted into 1-methyl-2-pyrrolidone (2x150 mL). Residual volatile organic solvents were distilled under vacuum. 4-Cyano-2,6-dimethoxyphenylboronic acid (17.5 g, 84.5 mmol) was added and the solution degassed. Tetrakis(triphenylphosphine) palladium(0) (3g, 2.7 mmol) and triethylamine (19.8 mL) were added and the reaction mixture heated to 70°C. The reaction was stirred at 70°C for at least 2 hours. Once complete by HPLC, the reaction mixture was transferred into another vessel and diluted with toluene (300 mL). 2M Hydrochloric acid (300 mL) was added and the mixture filtered. The organic layer was separated and washed with water (300 mL). The organic layer was filtered and washed through with toluene (90 mL). The toluene solution was stirred with Silicycle silica supported N-functionalised thiourea (11.7 g) for at least 15 hours. The silica was filtered and washed with toluene (30 mL). The solution was dried azeotropically then slowly added to a solution of hydrogen chloride in isopropanol (155 mL at 5M concentration) at 50°C. The reaction mixture was stirred at 50°C for 30 minutes until complete by HPLC. The mixture was cooled to 20°C and the product filtered off under vacuum and washed with toluene (60 mL). The solid was dried *in vacuo* at 40°C to yield the title compound as an off-white solid.

### 2-Bromo-5-hydroxybenzoic acid methyl ester

A solution of boron tribromide (18 mL, 190.5 mmol) in dichloromethane (18 mL) was added to a stirred suspension of 2-bromo-5-methoxybenzoic acid (from Wychem, 20 g, 86.6 mmol) in dichloromethane (200 mL) at -15 to -10°C. The yellow suspension was allowed to warm to 0°C. HPLC analysis showed complete consumption of acid. The reaction was quenched by addition of methanol (100 mL). The clear dark solution was stirred at reflux (approximately 45°C) for 12 hours, evaporated to near dryness, partitioned between water (400 mL) and ethyl acetate (500 mL), and separated. The organic solution was washed with brine (250 mL) and evaporated to yield the title compound as a cream solid.

### 2-Bromo-5-ethoxybenzoic acid methyl ester

A mixture of 2-bromo-5-hydroxybenzoic acid methyl ester (18.9 g, 81.8 mmol), potassium carbonate (34.4 g, 249.5 mmol) and ethyl iodide (13 mL, 163.7 mmol) in methyl isobutyl ketone (190 mL) was stirred at 70-75°C under nitrogen for 15 hours. HPLC analysis showed complete reaction to product. Water (100 mL) was added. The aqueous phase was removed and the methyl isobutyl ketone solution washed with brine (100 mL), then concentrated *in vacuo* to yield the title compound as an orange oil.

### 2-Bromo-5-ethoxybenzoic acid

A mixture of 2-bromo-5-ethoxybenzoic acid methyl ester (20.7 g, 79.9 mmol) and 10M sodium hydroxide (40 mL, 0.4 mol) in tetrahydrofuran (100 mL) was stirred at ambient for 18 hours. HPLC analysis showed complete consumption of starting material. The reaction was cooled to 0-5°C and acidified to pH1 with 5M hydrochloric acid. The organic phase was removed and the aqueous phase extracted will tertiary butyl methyl ether (40 mL). The organic phases were combined, washed with brine (50 mL) and concentrated *in vacuo* to yield the title compound as a cream solid.

### (S)-2-{[1-(2-Bromo-5-ethoxyphenyl)methanoyl]amino}-3-(4'-cyano-2',6'-dimethoxybiphenyl-4-yl)propionic acid methyl ester

To a suspension of (S)-2-amino-3-(4'-cyano-2',6'-dimethoxybiphenyl-4-yl)propionic acid methyl ester hydrochloride (5 g, 13.3 mmol) in tetrahydrofuran (100 mL) was added triethylamine (5.5 mL, 39.8 mmol). The suspension was stirred for 60 minutes at 20°C. A solution of 2-bromo-5-ethoxybenzoyl chloride¹ (3.4 g, 13 mmol) in toluene (10 mL) was added dropwise over 10 minutes maintaining the temperature at 0-5°C. The mixture was stirred at 0-5°C for at least 60 minutes. Once complete by HPLC the reaction mixture was washed sequentially with 2M hydrochloric acid (25 mL), 10%w/w aqueous sodium carbonate solution (25 mL) and 36%w/w brine (25 mL). The solvent was removed by distillation to give a cream solid. This was dissolved in tetrahydrofuran (75 mL) and stirred overnight with Silicycle silica supported N-functionalised thiourea (0.75 g). The silica was filtered and the tetrahydrofuran solution concentrated to approximately 30 mL. Water (75 mL) was added over 45 minutes. The slurry was stirred at ambient for 1 hour. Product was filtered, washed with water (30 mL) and dried *in vacuo* at 40°C to yield the title compound as an off-white solid.
¹ prepared from 2-bromo-5-ethoxybenzoic acid (3.2 g, 13 mmol) by standard procedures using thionyl chloride (3.3 equiv) in toluene (16 mL).

### (S)-2-{[1-(2-Bromo-5-ethoxyphenyl)methanoyl]amino}-3-(4'-cyano-2',6'-dimethoxybiphenyl-4-yl)propionic acid tetrahydrofuran solvate

A solution of (S)-2-{[1-(2-bromo-5-ethoxyphenyl)methanoyl]amino}-3-(4'-cyano-2',6'-dimethoxybiphenyl-4-yl)propionic acid methyl ester (5 g, 8.8 mmol) in tetrahydrofuran (25 mL) was cooled to 0-5°C. 2M Sodium hydroxide (4.5 mL, 9 mmol) was added dropwise keeping the temperature below 5°C. The reaction mixture was stirred at 0-5°C for at least 18 hours. Once complete by HPLC the mixture was diluted with water (50 mL) before being adjusted to pH1 with 2M hydrochloric acid (approximately 10 mL). The resulting suspension was aged for at least 30 minutes at 0-5°C before the solid was filtered off under vacuum and washed with water (20 mL). The product was then dried *in vacuo* at 40°C to yield the title compound as an off-white solid.

### (S)-2-{[1-(2-Bromo-5-ethoxyphenyl)methanoyl]amino}-3-(4'-cyano-2',6' dimethoxybiphenyl-4-yl)propionic acid (E1)

A suspension of (S)-2-{[1-(2-bromo-5-ethoxyphenyl)methanoyl]amino}-3-(4'-cyano-2',6'-dimethoxybiphenyl-4-yl)propionic acid tetrahydrofuran solvate (5 g) in methanol (50 mL) was heated to reflux and stirred to form a solution. The solution was cooled to 60°C and filtered. The solution was then cooled to 0°C over 1.5 hours. The resulting suspension was aged at 0°C for 2 hours. The solid was filtered off under vacuum and washed with cold methanol (5 mL). The product was dried *in vacuo* at 40°C to yield the title compound as a white crystalline solid.

### Example 2

### (S)-2-{[1-(2-Bromo-5-fluorophenyl)methanoyl]amino}-3-(4'-cyano-2',6'-dimethoxybiphenyl-4-yl)propionic acid (E2)

The title compound was prepared in an analogous manner to Preparation 11 and Example 1 using P13 and 2-bromo-5-fluorobenzoyl chloride (from Apollo). [M+H]⁺ at m/z 527, 529.

### Example 3

### (S)-2-([1-(2-Bromo-5-methoxyphenyl)methanoyl]amino)-3-(4'-cyano-2',6'-dimethoxybiphenyl-4-yl)propionic acid (E3)

### Example 3a

The title compound may be prepared in an analogous manner to Preparation 11 and Example 1 using P7 and 2-bromo-5-methoxybenzoyl chloride (from Avocado).

### Example 3b

Aqueous lithium hydroxide (0.5 M, 25 mL) was added with stirring to a solution of (S)-2-{[1-(2-bromo-5-methoxyphenyl)methanoyl]amino}-3-(4'-cyano-2',6'-dimethoxybiphenyl-4-yl)propionic acid ethyl ester (P12, 0.539 g, 0.95 mmol) in tetrahydrofuran (30 mL) at room temperature. Stirring was continued for 2 hours and the reaction then quenched with excess aqueous 10% citric acid. The mixture was then extracted with ethyl acetate and the organic layer washed with a further portion of citric acid and then water (x 2). The organic layer was evaporated to dryness and the resulting crude product purified by MDAP to provide the title compound as a white solid. [M+H]⁺ at m/z 539, 541.

### Example 4

### (S)-2-{[1-(2-Bromo-5-methylphenyl)methanoyl]amino}-3-(4'-cyano-2',6'-dimethoxybiphenyl-4-yl)propionic acid (E4)

The title compound was prepared in an analogous manner to Preparation 12 using P13 and 2-bromo-5-methylbenzoic acid (from Apin) and the resulting ethyl ester hydrolysed by the method of Example 3b. [M+H]⁺ at m/z 523, 525.

### Example 5

### (S)-2-{[1-(2-Bromo-5-chlorophenyl)methanoyl]amino}-3-[4'-cyano-2',6'-dimethoxybiphenyl-4-yl]propionic acid (E5)

The title compound was prepared from the corresponding ethyl ester in an analogous manner to that of Example 3b. The ethyl ester was prepared from P13 and 2-bromo-5-chlorobenzoic acid (from Lancaster) using the method of Preparation 12. [M-H]⁻ at m/z 541,543,545.

### Example 6

### (S)-2-{[1-(2,5-Dibromophenyl)methanoyl]amino}-3-[4'-cyano-2',6'-dimethoxybiphenyl-4-yl]propionic acid (E6)

The title compound was prepared from the corresponding ethyl ester in an analogous manner to that of Example 3b. The ethyl ester was prepared from P13 and 2,5-dibromobenzoic acid (from Lancaster) by the method of Preparation 12. [M-H]⁻ at m/z 585, 587, 589.

### Example 7

### (S)-2-{[1-(5-(iso-Propoxy)-2-bromophenyl)methanoyl]amino}-3-[4'-cyano-2',6'-dimethoxybiphenyl-4-yl]propionic acid (E7)

The title compound was prepared from the corresponding ethyl ester in an analogous manner to that of Example 3b. The ethyl ester was prepared from P13 and 5*-iso*propoxy-2-bromobenzoic acid (prepared analogously to the sequence Preparation 8 - Preparation 10 except for the use of iso-propylbromide in the alkylation step analogous to Preparation 9) by the method of Preparation 12. [M-H]⁻ at m/z 565, 567.

## Claims

1. A compound of formula (I) or a pharmaceutically acceptable salt, ester or solvate thereof : 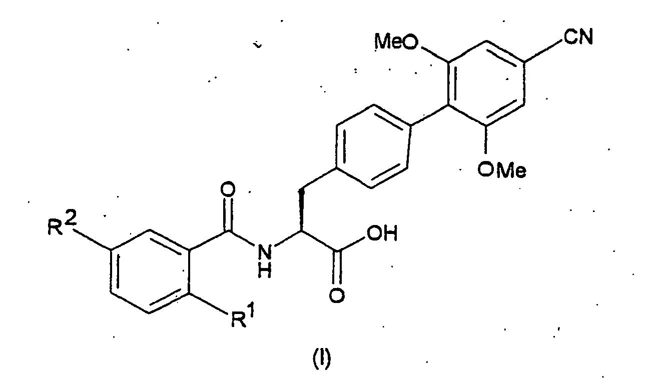 in which:
R¹ is bromo; and
R² is halogen, C₁₋₆alkyl or C₁₋₆alkoxy.

2. The compound according to claim 1 in which R² is halogen or C₁₋₆alkoxy.

3. The compound according to claim 2 in which R² is fluoro, methoxy or ethoxy.

4. The compound according to claim 1 which is selected from the group consisting of
(S)-2-{[1-(2-Bromo-5-methylphenyl)methanoyl]amino}-3-(4'-cyano-2',6'-dimethoxybiphenyl-4-yl)propionic acid;
(S)-2-{[(2-Bromo-5-chlorophenyl)methanoyl]amino}-3-[4'-cyano-2',6'-dimethoxybiphenyl-4-yl]propionic acid;
(S)-2-{[(2,5-Dibromophenyl)methanoyl]amino}-3-[4'-cyano-2',6'-dimethoxybiphenyl-4-yl]propionic acid;
(S)-2-{[(5-(*iso*-Propoxy)-2-bromophenyl)methanoyl]amino}-3-[4'-cyano-2',6'-dimethoxybiphenyl-4-yl]propionic acid;
or a pharmaceutically acceptable salt, ester or solvate thereof.

5. (S)-2-{[1-(2-Bromo-5-ethoxyphenyl)methanoyl]amino)-3-(4'-cyano-2',6'-dimethoxybiphenyl-4-yl)propionic acid or a pharmaceutically acceptable salt, ester or solvate thereof.

6. (*S*)-2-{[1-(2-Bromo-5-fluorophenyl)methanoyl]amino}-3-(4'-cyano-2',6'-dimethoxybiphenyl-4-yl)propionic acid or a pharmaceutically acceptable salt, ester or solvate thereof.

7. (*S*)-2-{[1-(2-Bromo-5-methoxyphenyl)methanoyl]amino}-3-(4'-cyano-2',6'-dimethoxybiphenyl-4-yl)propionic acid or a pharmaceutically acceptable salt, ester or solvate thereof.

8. A process for the preparation of a compound of formula (I) as defined in any of claims 1 to 4 which comprises hydrolyzing of a carboxylic acid ester derivative of formula (II): 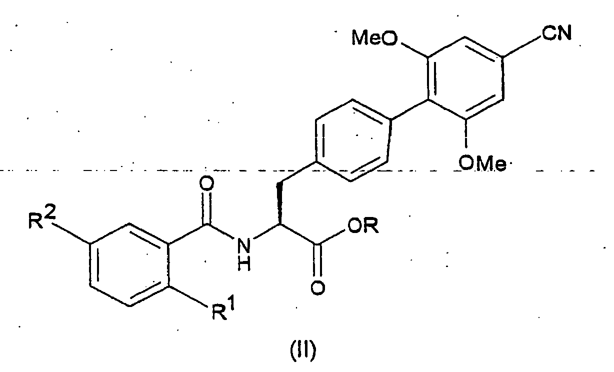 in which R¹ and R² are as defined in formula (I) and R is a C₁₋₆ alkyl group and optionally thereafter forming a Pharmaceutical acceptable salt, ester or solvate thereof.

9. A compound according to any one of claims 1 to 7 for use in therapy:

10. A pharmaceutical composition which comprises a therapeutically effective amount of a compound according to any one of claims 1 to 7 in admixture with a pharmaceutically acceptable carrier or diluent.

11. A pharmaceutical composition comprising a compound according to any one of claims 1 to 7 together with another therapeutically active agent

12. A use of a compound according to any one of claims 1 to 7 in the manufacture of a medicament for the treatment or prevention of a condition in which an inhibitor of α₄ integrin mediated cell adhesion is beneficial. wherein said condition is selected from the group consisting of rheumatoid arthritis (RA); asthma; allergic conditions such as rhinitis; adult respiratory distress syndrome; AIDS-dementia; Alzheimer's disease; cardiovascular diseases; thrombosis or harmful platelet aggregation; reocclusion following thrombolysis; reperfusion injury; skin inflammatory diseases such as psoriasis, eczema, contact dermatitis and atopic dermatitis; diabetes (e.g., insulin-dependent diabetes mellitus, autoimmune diabetes); multiple sclerosis; systemic lupus erythematosus (SLE); inflammatory bowel disease such as ulcerative colitis, Crohn's disease (regional enteritis) and pouchitis (for example, resulting after proctocolectomy and ileoanal anastomosis); diseases associated with leukocyte infiltration to the gastrointestinal tract such as Celiac disease, nontropical Sprue, enteropathy associated with seronegative arthropathies, lymphocytic or collagenous colitis, and eosinophilic gastroenteritis; diseases associated with leukocyte infiltration to other epithelial lined tissues, such as skin, urinary tract, respiratory airway, and joint synovium; pancreatitis; mastitis (mammary gland); hepatitis; cholecystitis; cholangitis or pericholangitis (bile duct and surrounding tissue of the liver); bronchitis; sinusitis; inflammatory diseases of the lung which result in interstitial fibrosis, such as hypersensitivity pneumonitis; collagen disease (in SLE and RA); sarcoidosis; osteoporosis; osteoarthritis; atherosclerosis; neoplastic diseases including metastasis of neoplastic or cancerous growth; wound (wound healing enhancement); certain eye diseases such as retinal detachment, allergic conjunctivitis and autoimmune uveitis; Sjogren's syndrome; rejection (chronic and acute) after organ transplantation; host vs. graft or graft vs. host diseases; intimal hyperplasia; arteriosclerosis (including graft arteriosclerosis after transplantation); reinfarction or restenosis after surgery such as percutaneous transluminal coronary angioplasty (PTCA) and percutaneous transluminal artery recanalization; nephritis; tumor angiogenesis; malignant tumor; multiple myeloma and myeloma-induced bone resorption; sepsis; and central nervous system injury such as stroke, traumatic brain injury and spinal cord injury and Meniere's disease.

13. The use according to claim 12, wherein said condition is inflammatory bowel disease or multiple sclerosis.

14. A compound of formula (II): 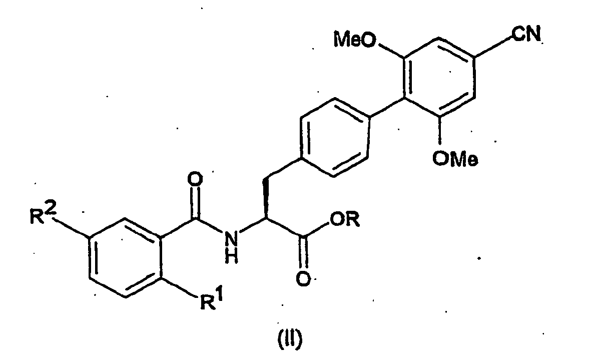 in which R¹ and R² are as defined in formula (I) as defined in any one of claims 1 to 4 and R is a, C₁₋₆ alkylgroup.

15. A compound according to claim 14 in which R² is C₁₋₆alkoxy or fluoro.

16. A compound of formula (III) or an acid addition salt thereof. 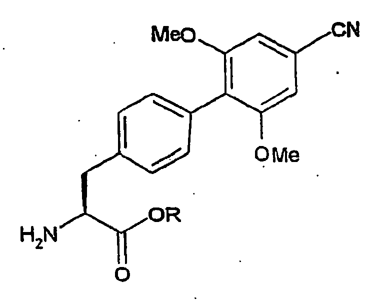 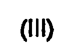 in which R is a C₁₋₆ alkyl group.

17. A compound of formula (VI):

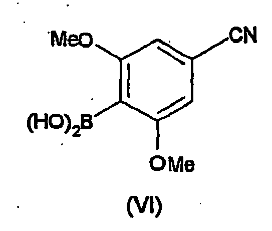

## Patentansprüche

1. Verbindung der Formel (I) oder ein pharmazeutisch verträgliches Salz, Ester oder Solvat davon: 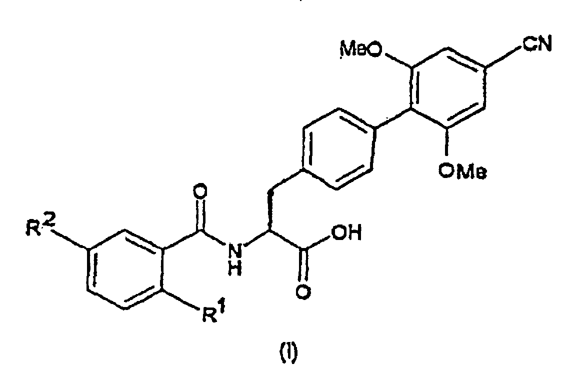 wobei:
R¹ für Brom steht; und
R² für Halogen, C₁₋₆-Alkyl oder C₁₋₆-Alkoxy steht.

2. Verbindung gemäß Anspruch 1, wobei R² für Halogen oder C₁₋₆-Alkoxy steht.

3. Verbindung gemäß Anspruch 2, wobei R² für Fluor, Methoxy oder Ethoxy steht.

4. Verbindung gemäß Anspruch 1, welche ausgewählt ist aus
(S)-2-{ [1-(2-Brom-5-methylphenyl)methanoyl]amino}-3-(4'-cyano-2',6'-dimethoxy-biphenyl-4-yl)propionsäure;
(S)-2-{ [(2-Brom-5-chlorphenyl)methanoyl]amino}-3-[4'-cyano-2',6'-dimethoxy-biphenyl-4-yl]propionsäure;
(S)-2-{[(2,5-Dibromphenyl)methanoyl]amino}-3-[4'-cyano-2',6'-dimethoxybiphenyl-4-yl]propionsäure;
(S)-2- ([(5(*iso*-Propoxy)-2-bromphenyl)methanoyl]amino}-3-[4'-cyano-2',6'-dimethoxy-biphenyl-4-yl]propionsäure;
oder ein pharmazeutisch verträgliches Salz, Ester oder Solvat davon.

5. (S)-2-{[1-(2-Brom-5-ethoxyphenyl)methanoyl]amino}-3-(4'-cyano-2',6'-dimethoxy-biphenyl-4-yl)propionsäure oder ein pharmazeutisch verträgliches Salz, Ester oder Solvat davon.

6. (S)-2-{[1-(2-Brom-5-fluorphenyl)methanoyl]amino}-3-(4'-cyano-2',6'-dimethoxy-biphenyl-4-yl)propionsäure oder ein pharmazeutisch verträgliches Salz, Ester oder Solvat davon.

7. (S)-2-{[1-(2-Brom-5-methoxyphenyl)methanoyl]amino}-3-(4'-cyano-2',6'-dimethoxy-biphenyl-4-yl)propionsäure oder ein pharmazeutisch verträgliches Salz, Ester oder Solvat davon.

8. Verfahren zur Herstellung einer Verbindung der Formel (I) wie in einem der Ansprüche 1 bis 4 definiert, umfassend die Hydrolyse eines Carbonsäureesterderivats der Formel (II): 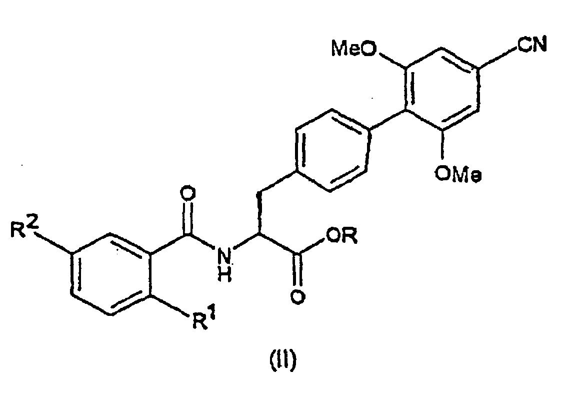 wobei R¹ und R² wie in Formel (I) definiert sind und R für einen C₁₋₆-Alkylrest steht, und gegebenenfalls nachfolgendes Bilden eines pharmazeutisch verträglichen Salzes, Esters oder Solvats davon.

9. Verbindung gemäß einem der Ansprüche 1 bis 7 zur Verwendung in der Therapie.

10. Arzneimittel, umfassend eine therapeutisch wirksame Menge einer Verbindung gemäß einem der Ansprüche 1 bis 7 in Beimischung mit einem pharmazeutisch verträglichen Träger oder Verdünnungsmittel.

11. Arzneimittel, umfassend eine Verbindung gemäß einem der Ansprüche 1 bis 7 zusammen mit einem anderen therapeutischen Wirkstoff.

12. Verwendung einer Verbindung gemäß einem der Ansprüche 1 bis 7 zur Herstellung eines Medikaments zur Behandlung oder Vorbeugung eines Zustandes, bei welchem ein Hemmstoff einer durch α₄-Integrin vermittelten Zelladhäsion vorteilhaft ist, wobei dieser Zustand ausgewählt ist aus rheumatoider Arthritis (RA); Asthma; allergischen Zuständen wie Rhinitis; Atemnotsyndrom der Erwachsenen; AIDS-Demenz; Alzheimer-Krankheit; kardiovaskulären Erkrankungen; Thrombose oder schädlicher Agglomeration der Thrombozyten; Reokklusion nach einer Thrombolyse; Reperfusionsverletzung; entzündlichen Erkrankungen der Haut wie Psoriasis, Ekzem, Kontaktdermatitis und atopischer Dermatitis; Diabetes (z.B. insulinabhängiger Diabetes, Immundiabetes); Multipler Sklerose; Lupus erythematodes visceralis (SLE); entzündlicher Darmerkrankung wie Colitis ulcerosa, Morbus Crohn (Enteritis regionalis) und Pouchitis (resultierend z.B. nach einer Proktokolektomie und Ileonalanastomose); Erkrankungen in Verbindung mit Leukozyteninfiltration im Magen-Darm-Trakt wie Zöliakie, einheimischer Sprue, Enteropathie in Verbindung mit seronegativen Arthropathien, lymphozytischer oder kollagener Colitis und eosinophiler Gastroenteritis; Erkrankungen in Verbindung mit Leukozyteninfiltration anderer mit Epithelien ausgekleideter Gewebe wie Haut, Harntrakt, Atemwege und Gelenkinnenhaut; Pankreatitis; Mastitis (Brustdrüse); Hepatitis; Cholezystitis; Cholangitis oder Pericholangitis (Gallengang und die Leber umgebendes Gewebe); Bronchitis; Sinusitis; entzündlichen Erkrankungen der Lunge, welche in interstitieller Fibrose resultieren, wie Hypersensitivitätspneumonitis; Kollagenose (bei SLA und RA); Sarkoidose; Osteoporose; Osteoarthritis; Artherosklerose; Neoplasie-Erkrankungen einschließlich Metastase bei Neoplasie- oder Krebswachstum; Wunden (Verbesserung der Wundheilung); bestimmten Augenerkrankungen wie Netzhautablösung, allergische Konjunktivitis und Autoimmun-Uveitis; Sjögren-Syndrom; Abstoßung (chronisch und akut) nach Organtransplantation; Wirt-Transplantat- oder Transplantat-Wirt-Reaktion; Intimahyperplasie; Arteriosclerosis (einschließlich Transplantat-Arteriosclerosis nach Transplantation); Reinfarkt oder Restenose nach einer Operation wie perkutaner transluminaler Koronarangioplastie (PTCA) und perkutaner transluminaler Arterienrekanalisation; Nierenentzündung; Tumorangiogenese; malignem Tumor; multiplem Myelom und Myelom-induzierter Knochenresorption; Sepsis; und Verletzung des zentralen Nervensystems wie Schlaganfall, traumatischer Hirnverletzung und Verletzung des Rückenmarks und Menière-Krankheit.

13. Verwendung gemäß Anspruch 12, wobei der Zustand eine entzündliche Darmerkrankung oder Multiple Sklerose ist.

14. Verbindung der Formel (II) 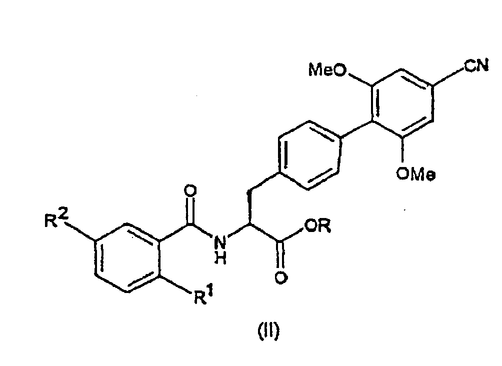 wobei R¹ und R² wie in einem der Ansprüche 1 bis 4 für Formel (I) definiert sind und R ein C₁₋₆-Alkylrest ist.

15. Verbindung gemäß Anspruch 14, wobei R² für C₁₋₆-Alkoxy oder Fluor steht.

16. Verbindung der Formel (III) oder ein Säureadditionssalz davon: 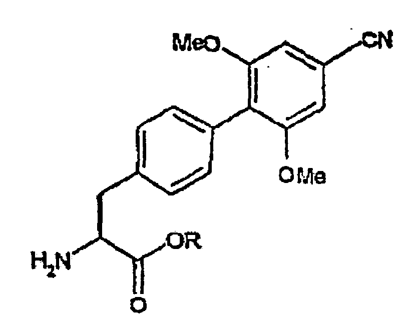 wobei R ein C₁₋₆-Alkylrest ist.

17. Verbindung der Formel (VI):

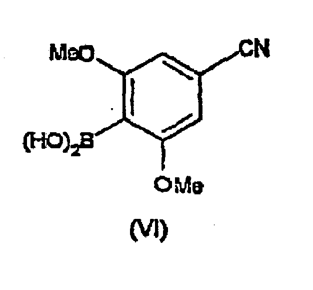

## Revendications

1. Composé de formule (I) ou un sel, ester ou solvate pharmaceutiquement acceptable de celui-ci : 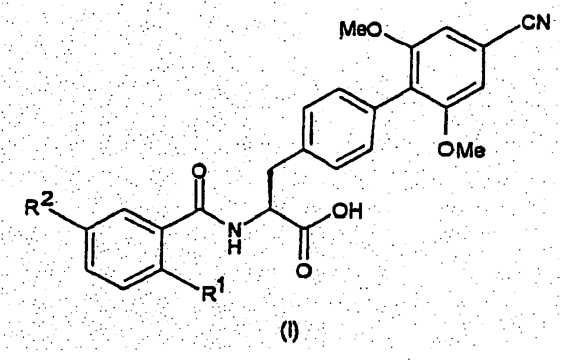 dans laquelle :
R¹ est bromo ; et
R² est un atome d'halogène, un alkyle en C_{1 à 6} ou un groupe alcoxy en C_{1 à 6·}

2. Composé selon la revendication 1, dans lequel R² est un atome d'halogène ou un groupe alcoxy en C_{1 à 6.}

3. Composé selon la revendication 2, dans lequel R² est fluoro, méthoxy ou éthoxy.

4. Composé selon la revendication 1, qui est choisi dans le groupe constitué de
l'acide (S)-2-{[1-(2-bromo-5-méthylphényl)méthanoyl]amino}-3-(4'-cyano-2',6'-diméthoxybiphényl-4-yl)propionique;
l'acide (S)-2-{[(2-bromo-5-chlorophényl)méthanoyl]amino}-3-[4'-cyano-2',6'-diméthoxy biphényl-4-yl]propionique ;
l'acide (S)-2-{[(2,5-dibromophényl)méthanoyl]amino}-3-[4'-cyano-2',6'-diméthoxy biphényl-4-yl]propionique ;
l'acide (S)-2-{[(5-(iso-propoxy)-2-bromophényl)méthanoyl]amino}-3-[4'-cyano-2',6'-diméthoxy biphényl-4-yl]propionique ;
ou un sel, ester ou solvate pharmaceutiquement acceptable de ceux-ci.

5. Acide (S)-2-{[1-(2-bromo-5-éthoxyphényl)méthanoyl]amino}-3-(4'-cyano-2',6'-diméthoxybiphényl-4-yl)propionique ou un sel, ester ou solvate pharmaceutiquement acceptable de celui-ci.

6. Acide (S)-2-{[1-(2-bromo-5-fluorophényl)méthanoyl)amino}-3-(4'-cyano-2',6'diméthoxybiphényl-4-yl)propionique ou un sel, ester ou solvate pharmaceutiquement acceptable de celui-ci.

7. Acide (S)-2-{[1-(2-bromo-5-méthoxyphényl)méthanoyl]amino}-3-(4'-cyano-2',6'-diméthoxybiphényl-4-yl)propionique ou un sel, ester ou solvate pharmaceutiquement acceptable de celui-ci.

8. Procédé pour la préparation d'un composé de formule (I) selon l'une quelconque des revendications 1 à 4, qui comprend une hydrolyse d'un dérivé d'ester d'acide carboxylique de formule (II) : 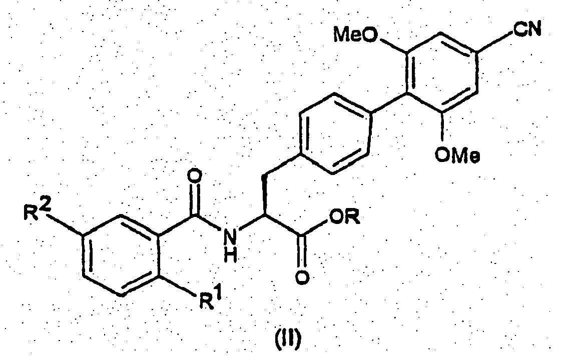 dans laquelle R¹ et R² sont tels que définis dans la formule (I) et R est un groupe alkyle en C_{1 à 6} formant facultativement par la suite un sel, ester ou solvate pharmaceutiquement acceptable de celui-ci.

9. Composé selon l'une quelconque des revendications 1 à 7 pour une utilisation en thérapie.

10. Composition pharmaceutique qui comprend une quantité thérapeutiquement efficace d'un composé selon l'une quelconque des revendications 1 à 7 en mélange avec un vecteur ou diluant pharmaceutiquement acceptable.

11. Composition pharmaceutique comprenant un composé selon l'une quelconque des revendications 1 à 7, conjointement avec un autre agent thérapeutiquement actif.

12. Utilisation d'un composé selon l'une quelconque des revendications 1 à 7 dans la fabrication d'un médicament pour le traitement ou la prévention d'une maladie dans laquelle un inhibiteur d'adhésion cellulaire médié par intégrine α₄ est bénéfique, dans lequel ladite maladie est choisie dans le groupe constitué de la polyarthrite rhumatoïde (RA) ; l'asthme ; les maladies allergiques telles que la rhinite ; le syndrome de détresse respiratoire de l'adulte ; la démence liée au SIDA ; la maladie d'Alzheimer ; les maladies cardiovasculaires ; la thrombose ou l'agrégation plaquettaire nocive ; la réocclusion après thrombolyse ; la lésion de reperfusion; les maladies inflammatoires de la peau telles que le psoriasis, l'eczéma, l'eczéma de contact et la dermite atopique, le diabète (par exemple, diabète de type I, diabète auto-immun) ; la sclérose en plaques ; le lupus érythémateux disséminé (SLE) ; l'affection abdominale inflammatoire telle que la rectocolite hémorragique, la maladie de Crohn et la pochite (par exemple, résultant d'une proctocolectomie et une anastomose iléo-anale) ; les maladies associées à une infiltration leucocytaire dans le tractus gastro-intestinal telles que la maladie coeliaque, l'entéropathie associée à des arthropathies séronégatives, la colite collagène ou lymphocytaire, et la gastro-entérite éosinophile ; les maladies associées à l'infiltration leucocytaire à d'autres tissus épithéliaux tapissés, tels que la peau, le tractus urinaire, les voies respiratoires et la synoviale articulaire ; la pancréatite ; la mastite (glande mammaire) ; l'hépatite ; la cholécystite ; l'angiocholite ou la périangiocholite (voie biliaire principale et tissu environnant du foie) ; la bronchite ; la sinusite ; les maladies inflammatoires du poumon qui conduisent à la fibrose interstitielle, telles que l'alvéolite allergique extrinsèque ; la maladie du collagène (dans SLE et RA) ; la sarcoïdose ; l'ostéoporose ; l'arthrose ; l'athérosclérose ; les maladies néoplasiques comprenant la métastase de croissance néoplasique ou cancéreuse ; une plaie (amélioration de cicatrisation des plaies) ; certaines maladies de l'oeil telles que le décollement rétinien, la conjonctivite allergique et l'uvéite auto-immune ; le syndrome de Sjögren ; le rejet (chronique et aigu) après transplantation d'organe ; les maladies de l'hôte contre le greffon ou de greffon contre l'hôte ; l'hyperplasie intime ; l'artériosclérose (y compris l'artériosclérose de greffe après transplantation) ; le réinfarctus ou la resténose après chirurgie telle que l'angioplastie coronarienne transluminale percutanée (PTCA) et la recanalisation artérielle transluminale percutanée ; la néphrite ; l'angiogenèse tumorale ; la tumeur maligne ; le myélome multiple et la résorption osseuse induite par myélome ; la sepsie et la lésion du système nerveux central telle que l'accident vasculaire cérébral, la lésion cérébrale traumatique et la lésion de la moelle épinière et la maladie de Ménière.

13. Utilisation selon la revendication 12, dans laquelle ladite maladie est l'affection abdominale inflammatoire ou la sclérose en plaques.

14. Composé de formule (II) : 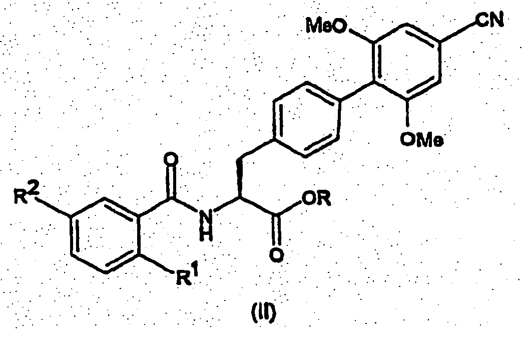 dans laquelle R¹ et R² sont tels que définis dans la formule (I) telle que définie selon l'une quelconque des revendications 1 à 4 et R est un groupe alkyle en C_{1 à 6}.

15. Composé selon la revendication 14, dans lequel R² est un alcoxy en C_{1 à 6} ou fluoro.

16. Composé de formule (III) ou un sel d'addition acide de celui-ci ; 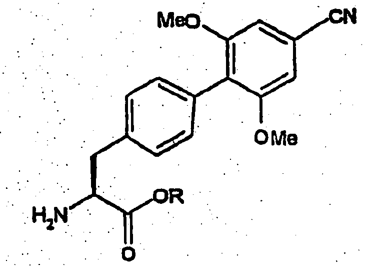 dans laquelle R est un groupe alkyle en C_{1 à 6}.

17. Composé de formule (VI) :
